# EUROPÄISCHE PATENTSCHRIFT

(11) **EP 0 248 231 B1**
(45) Veröffentlichungstag und Bekanntmachung des Hinweises auf die Patenterteilung: **28.07.1993**
(21) Anmeldenummer: 87106649.4
(22) Anmeldetag: 07.05.1987
(51) Int. Cl.: C07K 5/10, C07K 7/00, A61K 37/02, G01N 33/68

(54) **Pharmakologisch aktive Peptide**
Pharmacologically active peptides
Peptides à activité pharmacologique

(30) Priorität: 31.05.1986 DE 3618407
(43) Veröffentlichungstag der Anmeldung: 09.12.1987
(73) Patentinhaber: Brantl, Victor, Dr. med., Dipl.-Chem., D-7846 Schliengen (DE)
(72) Erfinder: Brantl, Victor, Dr. med., Dipl.-Chem., D-7846 Schliengen (DE)
(74) Vertreter: Schacht, Wilhelm, Dr. jur.

(56) Entgegenhaltungen:
- EP-A- 0 019 829
- EUROPEAN JOURNAL OF PHARMACOLOGY, Band 125, 1986, Seiten 309-310, Elsevier, Amsterdam, NL; V. BRANTL et al.: "Novel opioid peptides defived from hemoglobin: Hemorphins"
- J. BIOCHEM, Band 234, 1. März 1986, Seiten 441-447, London, GB; N. YOSHIOKA et al.: "Localization of the antigenic sites of the beta-chain in three host species by synthetic overlapping peptides representing the entire chain"
- BIOCHIMICA ET BIOPHYSICA ACTA, Band 625, 1980, Seiten 266-273, Elsevier/North-Holland Biomedical Press; R.C.C. CHANG et al.: "Isolation and structure of several peptides from porcine hypothalami"
- Eur. J. Pharm. Vol. 125 (1986), p.310
- Life Sciences Vol. 33, (1983) , p. 137-140

## Beschreibung

Die Offenlegungsschrift DE 33 40 208 A1 beschreibt Peptide der allgemeinen Formel:

X-A-B-C-Trp-D-Y, (I)

die gemäß den darin enthaltenen Ansprüchen 1 und 2 zu beispielsweise folgenden Verbindungen konkretisiert werden können:

Phe-Pro-Trp-D (II) D = Val, Leu, Met, Met(O), Ile, Phe

Tyr-Pro-Trp-D (III) D = Val, Leu, Met, Met(O), Ile, Phe

Phe-Pro-Pro-Trp-D (IV) D = Val, Leu, Met, Met(O), Ile, Phe

Tyr-Pro-Pro-Trp-D (V) D = Val, Leu, Met, Met(O), Ile, Phe

Diese Peptide wurden ursprünglich aus der Froschhaut isoliert und wegen der enthaltenen Aminosäuren Tryptophan als "Tryptophylline" bezeichnet. Die Charakterisierung der Tryptophylline ergab u.a. die folgenden Strukturmerkmale:

Phe-Pro-Pro-Trp-Val (VI)

Phe-Pro-Pro-Trp-Met (VII)

Phe-Pro-Pro-Trp-Leu (VIII)

<Glu-Pro-Trp-Met-NH₂ (IX).

Vergleiche hierzu: P.C. Montecucchi et al., Int. J. Peptide, Protein Res. 23, 276-281, 1984, Eingang bei der Schriftleitung am 31.03.83. Diese Arbeit war der Ausgangspunkt der bereits erwähnten Offenlegungsschrift DE 33 40 208 Al und beschreibt den beim Anmeldezeitpunkt vorhandenen Wissensstand. So ist der Seite 280 - rechte Spalte, letzter Absatz - der Veröffentlichung von P.C. Montecucchi et al. (1984) zu entnehmen, daß die in der DE 33 40 208 A1 beschriebenen Verbindungen an isolierten Organpräparaten (glatter Muskel) nicht aktiv sind.

Die genannte Offenlegungsschrift, die Literatur von Montecucchi et al. (1984) und auch spätere Arbeiten (Gozzini et al., Int. J. Peptide, Protein Res. 25, 323-329, 1985) bestätigen die Inaktivität der Tryptophylline an üblichen glatten Muskelpräparaten und lehren gezielt davon weg, opiatartige und somit an isolierten Organpräparaten aktive Verbindungen innerhalb der Peptide aus der Gruppe der Tryptophylline zu suchen. Überraschenderweise wurde nunmehr gefunden und erkannt, daß die Peptide gemäß der hier vorliegenden Erfindung - insbesondere der Verbindung Tyr-Pro-Trp-Thr (X) und die am C-terminalen Ende verlängerten Peptide ähnlicher Struktur - opiatartig wirken und an isolierten Organpräparaten aktiv sind.

Aufgabe der vorliegenden Erfindung ist es, neuartige Peptide zu schaffen, die opiatartig wirken und an isolierten Organpräparaten wirksam sind.

Diese Aufgabe der Erfindung ist dadurch gelöst, daß die pharmakologisch aktiven Peptide die allgemeine Formel: 
Tyr-Pro-Trp-Thr-T
Tyr-Pro-Trp-Thr-A-T
Tyr-Pro-Trp-Thr-A-B-T
Tyr-Pro-Trp-Thr-A-B-C-T
Tyr-Pro-Trp-Thr-A-B-C-D-T
Tyr-Pro-Trp-Thr-A-B-C-D-E-T
Tyr-Pro-Trp-Thr-A-B-C-D-E-F-T
aufweisen,
wobei Tyr gleich der Aminosäure Tyrosin, das auch als N-terminales L-Tyrosin der allgemeinen Formel 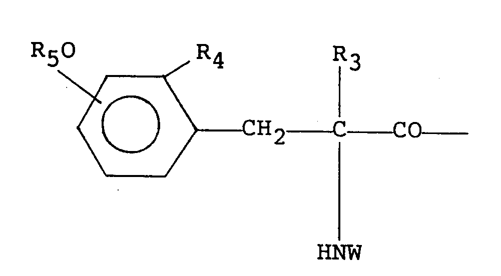
vorliegt, ist, wobei im einzelnen bedeutet:
- R₃: Wasserstoff oder eine Alkylgruppe mit 1 bis 4 C-Atomen,
- R₄: Wasserstoff oder zusammen mit R₃ für eine Äthylbrücke,
- R₅: Wasserstoff, eine Alkylgruppe mit 1 bis 4 C-Atomen oder eine R₆CO-Gruppe,
- R₆: einen gesättigten oder ungesättigten, geradkettigen oder verzweigten Alkylrest mit 1 bis 17 C-Atomen, einen Phenylrest oder einen Phenylalkylrest mit 7 bis 12 C-Atomen, wobei die Phenylreste durch 1 oder 2 Substituenten aus der Reihe Halogen, Alkyl mit 1 bis 4 C-Atomen oder Alkoxy mit 1 bis 4 C-Atomen substituiert sein können, wobei die R₅O-Gruppe sich in meta- oder para-Stellung zum 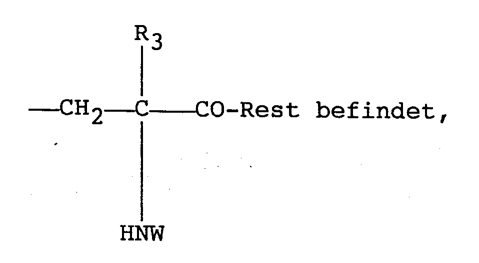
- W: Wasserstoff, Alkyl mit 1 bis 5 C-Atomen Alkenyl mit 3 bis 5 C-Atomen, Cyclopropylmethyl, Cyclobutylmethyl, R₆CO-, H-Val, H-Val-Val, H-Arg, H-Lys, H-Ile, H-Tyr, H-Phe oder D-Val;
Pro gleich der Aminosäure Prolin, das auch in der allgemeinen Formel 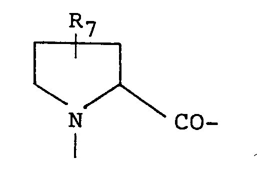
vorliegt, ist, wobei im einzelnen bedeutet:
- R₇: Wasserstoff, eine Hydroxygruppe, eine Alkyl- oder Alkoxygruppe mit 1 oder 4 C-Atomen,
- am Stickstoff eine Alkyl- oder Alkoxygruppe mit 1 bis 4 C-Atomen gebunden ist,
- eine oder mehrere Ketogruppen in den Ring eingeführt sind,
Trp gleich der Aminosäure Tryptophan, Thr gleich der Aminosäure Threonin. A, B, C, D, E und F können jede beliebige Aminosäure der D- oder L-Form sein, wobei das Nonapeptid Val-Val-Tyr-Pro-Trp-Thr-Gln-Arg-Phe ausgeschlossen ist; T steht für OH, OR, NH₂, NHR, NR₂ oder NHNHR', wobei R gegebenenfalls die folgende Bedeutung hat: Substit. lineares oder verzweigtes C₁₋₁₀-Alkyl, Adamantyl, C₁₋₁₀-Cycloalkyl oder C₆₋₈-Aralkyl, zweckmäßigerweise Phenyl, Benzyl oder Phenyläthyl bedeutet und R' Wasserstoff, lineares oder verzweigtes C₁₋₁₀-Alkyl, Cycloalkyl oder C₆₋₈-Aralkyl, lineares, verzweigtes oder cyclisches aliphat. C₁₋₁₆-Acyl, gegebenenfalls durch OH, NH₂, C₁₋₁₄-Alkoxy oder Halogen substituiert, aromatisches Acyl, gegebenenfalls durch OH, NH₂, Halogen oder C₁₋₄-Alkoxy substituiert; lineares verzweigtes oder cyclisches C₃₋₁₁ aliphatisches Urethan darstellt und deren pharmazeutische annehmbaren Salze.

Nach einer Weiterbildung der Erfindung sind die Peptide dadurch gekennzeichnet, daß
- A =: Gln, Arg, Glu, Ser, Thr, Pro, Phe oder Tyr
- B =: Arg
- C =: Phe
- D =: Phe
- E =: Glu oder Asp
- F =: Ser
ist.

Die Aminosäure in der Position 2 (Pro²) kann vorteilhafterweise durch eine Aminosäure in der D-Konfiguration ersetzt sein. Die D-Aminosäure an Position 2 des Peptids bewirkt eine Steigerung der opiatartigen Wirkung und/oder eine Stabilisierung der Peptide gegen Abbau durch proteolytische Enzyme im Blut (enzymatische Stabilität). Besonders vorteilhaft ist es, wenn das Threonin (Thr⁴) in der D-Form vorliegt.

Bei den erfindungsgemäßen Peptiden kann die Aminosäure Prolin als Dehydroaminosäure vorliegen.

Nach einer Weiterbildung der Erfindung liegt das Tryptophan in der 3. Aminosäure-Position (Trp³) in der D-Form vor.

Bevorzugte erfindungsgemäße Peptide weisen die nachfolgenden Strukturen auf:

Val-Tyr-Pro-Trp-Thr
Tyr-Pro-Trp-Thr
Tyr-Pro-Trp-Thr-Gln
Tyr-Pro-Trp-Thr-NH2
Tyr-D-Ala-Trp-Thr-OH
Tyr-D-Ala-Trp-Thr-NH2
Tyr-D-Ala-Trp-D-Thr-Tyr-OH
Tyr-D-Ala-Trp-D-Thr-Phe-OH

Die erfindungsgemäßen Peptide können nach Kopplung an Thyreoglobulin oder andere makromolekulare Eiweißkörper als Antigene zur Erzeugung von Antikörpern im Säugetierorganismus verwendet werden.

Hierbei wird das Peptid in üblicher Weise mit Carbodiimid an das Makromolekül gekoppelt und intracutan in die Rücken- und Bauchhaut von Kaninchen injiziert.

Die so gewonnenen Antikörper können zur Bestimmung der erfindungsgemäßen Peptide in Körpergeweben und -flüssigkeiten verwendet werden.
Dies kann sowohl zur Bestimmung von exogen zugeführten wie auch endogen entstandener Peptide verwendet werden (Diagnostik).

Die erfindungsgemäßen Peptide und/oder deren Derivate und/oder deren Säureadditionssalze und/oder deren Metailkomplexe können in Arzneimitteln enthalten sein.

Die Arzneimittel können insbesondere als Antitussiva, Antidiarrhoika, Analgetika, Antipsychotika, zentrale und periphere Durchblutungsförderer, Tranquilizer und Wundheilungsstoffe Verwendung finden.

### Beispiel

1) Die Synthese zweier erfindungsgemäßer Peptide H-Tyr-Pro-Trp-Thr-OH und H-Tyr-Pro-Trp-Thr-Gln-OH erfolgte mit Hilfe eines in der Peptidchemie üblichen Syntheseapparates (Fa. Applied Biosystems, Modell 430 A) unter Benutzung der Fmoc-Methode (Fmoc = 9-fluorenylmethoxycarbonyl), so, wie von Meienhofer et al. im International Journal of Peptide Protein Research, 13, Seiten 35-42, 1979, beschrieben; die Aminosäuren wurden von der Firma Novabiochem, Schweiz, bezogen. Die Herstellung der anderen erfindungsgemäßen Peptide erfolgte analog dem Beispiel. Die synthetischen Peptide wurden dann mittels einer "reversed phase, C18" Hochdruckflüssigkeitschromatographie (HPLC) gereinigt, so wie von Brantl in "HPLC in Protein and Peptide Chemistry" (Eds. F. Lottspeich, A. Henschen, K.P. Hupe), Walter de Gruyter, Berlin, Seiten 365-384, 1981, beschrieben. Die pharmakologisch aktiven Fraktionen wurden nach saurer Hydrolyse auf ihre Aminosäurezusammensetzung geprüft und ergaben die richtige molare Zusammensetzung der erwarteten Aminosäurezusammensetzung; Methodik: Lottspeich et al., Hoppe-Seyler's Z. Physiol. Chem. 361, 1835-1839, 1980.
2) Pharmakologische Wirkungen zweier erfindungsgemäßer Peptide (H-Tyr-Pro-Trp-Thr-OH und H-Tyr-Pro-Trp-Thr-Gln-OH).

Die beiden Substanzen zeigen spezifische opiatartige Wirkungen am elektrisch stimulierten plexus myentericus/ Längsmuskel-Präparat des Meerschweinchenileums (GPI), Methode: Schulz und Goldstein, J. Pharmacol. Exptl. Ther. 183, 400, 1972. Die Ergebnisse sind in Tabelle 1 aufgeführt.

| Substanz | GPI |
|---|---|
| Tyr-Pro-Trp-Thr | 45,2 |
| Tyr-Pro-Trp-Thr-Gln | 46,1 |
| Normorphin | 0,1 |

Tabelle 1. Opiatartige Wirkungen zweier erfindungsgemäßer Peptide; die Zahlen geben diejenige Konzentration (µM) an, die notwendig ist, die elektrisch induzierte Kontraktion des Meerschweinchen-Darm-Präparates (GPI) um 50 % zu hemmen (IC₅₀-Wert). Die Zahlen sind Mittelwerte aus 4-6 Bestimmungen; die Standardabweichung von den Mittelwerten ist kleiner als 14 %. Die Hemmungen des GPI sind mit dem spezifischen Opioid-Antagonisten Naloxon aufhebbar bzw. bei Vorbehandlung blockierbar. (Endkonzentration des Naloxons im Organbad 0,5 µM).

Opiatartige Wirkungen können auch nach intracerebroventriculärer Injektion bei Ratten beobachtet werden. Hierbei wird so vorgegangen, wie von Brantl et al., Life Sciences, 28, 1903-1909, 1981, beschrieben wurde. Als wirksame analgetische Dosis erwiesen sich 350 bis 500 µg der o.g. Peptide; die analgetische Wirkung ist mit Naloxon, 10 mg pro kg Körpergewicht (intraperitoneal), aufhebbar bzw. bei Vorbehandlung mit Naloxon blockierbar.

Die Gewinnung von Antikörpern gegen die beiden synthetischen Peptide erfolgte genau so, wie für die ß-Casomorphine von U. Hamel et al. im Journal of Dairy Research, 52, 139-148, 1985, beschrieben. Kurz skizziert: Die beiden Peptide werden mittels Carbodiimid an Thyroglobulin gekoppelt und intracutan in die Rücken und Bauchhaut von Kaninchen injiziert. Als markierte Substanzen werden ¹²⁵I-Tyr-Pro-Trp-Thr-OH und ¹²⁵I-Tyr-Pro-Trp-Thr-Gln-OH verwendet; weiteres Vorgehen, genau wie bei Hamel et al., 1985, Seite 141 ff., unter "Radioimmunoassays" beschrieben.

## Patentansprüche (Patentansprüche für folgende(n) Vertragsstaat(en): BE, CH, DE, FR, GB, IT, LI, LU, NL, SE)

1. Pharmakologisch aktive Peptide der allgemeinen Formel: 
Tyr-Pro-Trp-Thr-T
Tyr-Pro-Trp-Thr-A-T
Tyr-Pro-Trp-Thr-A-B-T
Tyr-Pro-Trp-Thr-A-B-C-T
Tyr-Pro-Trp-Thr-A-B-C-D-T
Tyr-Pro-Trp-Thr-A-B-C-D-E-T
Tyr-Pro-Trp-Thr-A-B-C-D-E-F-T
 wobei Tyr gleich der Aminosäure Tyrosin, das auch als N-terminales L-Tyrosin der allgemeinen Formel 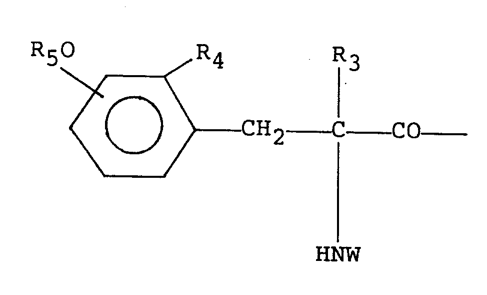 vorliegt, ist, wobei im einzelnen bedeutet:
R₃ Wasserstoff oder eine Alkylgruppe mit 1 bis 4 C-Atomen,
R₄ Wasserstoff oder zusammen mit R₃ für eine Äthylbrücke,
R₅ Wasserstoff, eine Alkylgruppe mit 1 bis 4 C-Atomen oder eine R₆CO-Gruppe,
R₆ einen gesättigten oder ungesättigten, geradkettigen oder verzweigten Alkylrest mit 1 bis 17 C-Atomen, einen Phenylrest oder einen Phenylalkylrest mit 7 bis 12 C-Atomen, wobei die Phenylreste durch 1 oder 2 Substituenten aus der Reihe Halogen, Alkyl mit 1 bis 4 C-Atomen oder Alkoxy mit 1 bis 4 C-Atomen substituiert sein können, wobei die R₅O-Gruppe sich in meta- oder para-Stellung zum 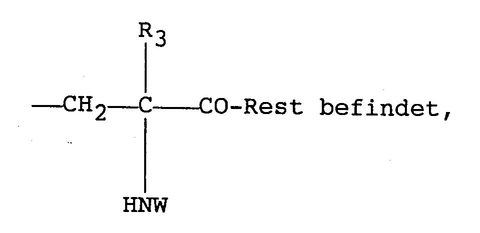
W Wasserstoff, Alkyl mit 1 bis 5 C-Atomen, Alkenyl mit 3 bis 5 C-Atomen, Cyclopropylmethyl, Cyclobutylmethyl, R₆CO-, H-Val, H-Val-Val, H-Arg, H-Lys, H-Ile, H-Tyr, H-Phe oder D-Val;
Pro gleich der Aminosäure Prolin, das auch in der allgemeinen Formel 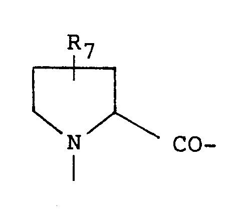 vorliegt, ist, wobei im einzelnen bedeutet:
R₇ Wasserstoff, eine Hydroxygruppe, eine Alkyl-oder Alkoxygruppe mit 1 oder 4 C-Atomen
- am Stickstoff eine Alkyl- oder Alkoxygruppe mit 1 bis 4 C-Atomen gebunden ist,
- eine oder mehrere Ketogruppen in den Ring eingeführt sind;
Trp gleich der Aminosäure Tryptophan, Thr gleich der Aminosäure Threonin. A, B, C, D, E und F können jede beliebige Aminosäure der D- oder L-Form sein, wobei das Nonapeptid Val-Val-Tyr-Pro-Trp-Thr-Gln-Arg-Phe ausgeschlossen ist;
T steht für OH, OR, NH₂, NHR, NR₂ oder NHNHR', wobei R gegebenenfalls die folgende Bedeutung hat: Substit. lineares oder verzweigtes C₁₋₁₀-Alkyl, Adamantyl, C₁₋₁₀-Cycloalkyl oder C₆₋₈-Aralkyl, zweckmäßigerweise Phenyl, Benzyl oder Phenyläthyl bedeutet und R' Wasserstoff, lineares oder verzweigtes C₁₋₁₀-Alkyl, Cycloalkyl oder C₆₋₈-Aralkyl, lineares, verzweigtes oder cyclisches aliphat. C₁₋₁₆-Acyl, gegebenenfalls durch OH, NH₂, C₁₋₁₄-Alkoxy oder Halogen substituiert, aromatisches Acyl, gegebenenfalls durch OH, NH2, Halogen oder C₁₋₄-Alkoxy substituiert; lineares verzweigtes oder cyclisches C₃₋₁₁aliphatisches Urethan darstellt und deren pharmazeutisch annehmbaren Salze.

2. Pharmakologisch aktive Peptide nach Anspruch 1, dadurch gekennzeichnet, daß
A = Gln, Arg, Glu, Ser, Thr, Pro, Phe oder Tyr
B = Arg
C = Phe
D = Phe
E = Glu oder Asp
F = Ser
ist.

3. Pharmakologisch aktive Peptide nach Ansprüchen 1 und 2, dadurch gekennzeichnet, daß Prolin in Position 2 durch D-Ala² oder D-Pro² ersetzt ist.

4. Pharmakologisch aktive Peptide nach Ansprüchen 1 bis 3, dadurch gekennzeichnet, daß Thr⁴ durch eine beliebige Aminosäure ersetzt ist, außer Val, Met(O), Ile, Phe und Pro.

5. Pharmakologisch aktive Peptide nach Ansprüchen 1 bis 4, dadurch gekennzeichnet, daß die Aminosäure Prolin als Dehydroaminosäure vorliegt.

6. Pharmakologisch aktive Peptide nach Ansprüchen 1 bis 5, dadurch gekennzeichnet, daß das Tryptophan in der 3. Aminosäureposition des Peptids in der D-Form vorliegt.

7. Pharmakologisch aktive Peptide nach Ansprüchen 1 bis 6, dadurch gekennzeichnet, daß das Threonin in der 4. Position in der D-Form vorliegt.

8. Pharmakologisch aktive Peptide, dadurch gekennzeichnet, daß die Peptide folgende Strukturen aufweisen:

Val-Tyr-Pro-Trp-Thr
Tyr-Pro-Trp-Thr
Tyr-Pro-Trp-Thr-Gln
Tyr-Pro-Trp-Thr-NH2
Tyr-D-Ala-Trp-Thr-OH
Tyr-D-Ala-Trp-Thr-NH2
Tyr-D-Ala-Trp-D-Thr-Tyr-OH
Tyr-D-Ala-Trp-D-Thr-Phe-OH

9. Pharmakologisch aktive Peptide nach Ansprüchen 1 bis 8, dadurch gekennzeichnet, daß diese nach Kopplung an Thyreoglobulin oder andere makromolekulare Eiweißkörper als Antigene zur Erzeugung von Antikörpern im Säugetierorganismus verwendet werden.

10. Pharmakologisch aktive Peptide nach Ansprüchen 1 bis 9, dadurch gekennzeichnet, daß Antikörper gegen die erfindungsgemäßen Peptide zur Bestimmung derselben in Körpergeweben und -flüssigkeiten verwendet werden.

11. Arzneimittel und Tierarzneimittel, dadurch gekennzeichnet, daß diese in den Ansprüchen 1 bis 10 charakterisierten pharmakologisch aktiven Peptide und/oder deren Derivate und/oder deren Säureadditionssalze und/oder deren Metallkomplexe enthalten.

## Patentansprüche (Patentansprüche für folgende(n) Vertragsstaat(en): AT, ES, GR)

1. Verfahren zur Herstellung pharmakologisch aktiver Peptide, dadurch gekennzeichnet, daß Peptide der allgemeinen Formel: 
Tyr-Pro-Trp-Thr-T
Tyr-Pro-Trp-Thr-A-T
Tyr-Pro-Trp-Thr-A-B-T
Tyr-Pro-Trp-Thr-A-B-C-T
Tyr-Pro-Trp-Thr-A-B-C-D-T
Tyr-Pro-Trp-Thr-A-B-C-D-E-T
Tyr-Pro-Trp-Thr-A-B-C-D-E-F-T
 wobei Tyr gleich der Aminosäure Tyrosin, das auch als N-terminales L-Tyrosin der allgemeinen Formel 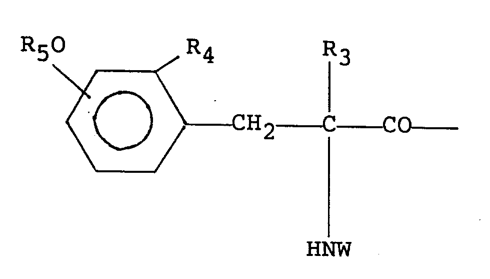 vorliegt, ist, wobei im einzelnen bedeutet:
R₃ Wasserstoff oder eine Alkylgruppe mit 1 bis 4 C-Atomen,
R₄ Wasserstoff oder zusammen mit R₃ für eine Äthylbrücke,
R₅ Wasserstoff, eine Alkylgruppe mit 1 bis 4 C-Atomen oder eine R₆CO-Gruppe,
R₆ einen gesättigten oder ungesättigten, geradkettigen oder verzweigten Alkylrest mit 1 bis 17 C-Atomen, einen Phenylrest oder einen Phenylalkylrest mit 7 bis 12 C-Atomen, wobei die Phenylreste durch 1 oder 2 Substituenten aus der Reihe Halogen, Alkyl mit 1 bis 4 C-Atomen oder Alkoxy mit 1 bis 4 C-Atomen substituiert sein können, wobei die R₅O-Gruppe sich in meta- oder para-Stellung zum 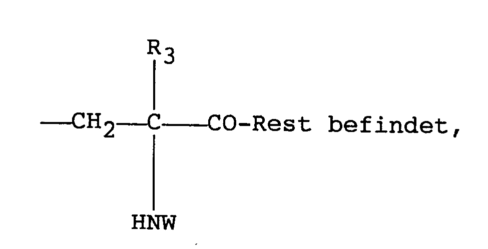
W Wasserstoff, Alkyl mit 1 bis 5 C-Atomen, Alkenyl mit 3 bis 5 C-Atomen, Cyclopropylmethyl, Cyclobutylmethyl, R₆CO-, H-Val, H-Val-Val, H-Arg, H-Lys, H-Ile, H-Tyr, H-Phe oder D-Val;
Pro gleich der Aminosäure Prolin, das auch in der allgemeinen Formel 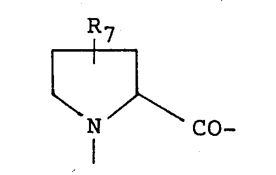 vorliegt, ist, wobei im einzelnen bedeutet:
R₇ Wasserstoff, eine Hydroxygruppe, eine Alkyl-oder Alkoxygruppe mit 1 oder 4 C-Atomen
- am Stickstoff eine Alkyl- oder Alkoxygruppe mit 1 bis 4 C-Atomen gebunden ist,
- eine oder mehrere Ketogruppen in den Ring eingeführt sind;
Trp gleich der Aminosäure Tryptophan, Thr gleich der Aminosäure Threonin. A, B, C, D, E und F können jede beliebige Aminosäure der D- oder L-Form sein, wobei das Nonapeptid Val-Val-Tyr-Pro-Trp-Thr-Gln-Arg-Phe ausgeschlossen ist;
T steht für OH, OR, NH₂, NHR, NR₂ oder NHNHR', wobei R gegebenenfalls die folgende Bedeutung hat: Substit. lineares oder verzweigtes C₁₋₁₀-Alkyl, Adamantyl, C₁₋₁₀-Cycloalkyl oder C₆₋₈-Aralkyl, zweckmäßigerweise Phenyl, Benzyl oder Phenyläthyl bedeutet und R' Wasserstoff, lineares oder verzweigtes C₁₋₁₀-Alkyl, Cycloalkyl oder C₆₋₈-Aralkyl, lineares, verzweigtes oder cyclisches aliphat. C₁₋₁₆ -Acyl, gegebenenfalls durch OH, NH₂, C₁₋₁₄-Alkoxy oder Halogen substituiert, aromatisches Acyl, gegebenenfalls durch OH, NH₂, Halogen oder C₁₋₄-Alkoxy substituiert; lineares verzweigtes oder cyclisches C₃₋₁₁aliphatisches Urethan darstellt und deren pharmazeutisch annehmbaren Salze, die jeweiligen Aminosäuren mit Hilfe eines in der Peptidchemie üblichen Syntheseapparates unter Benutzung der 9-Fluor-enylmethoxycarbonyl-Methode gekoppelt werden und anschließend mittels einer reversed phase C 18- Hochdruckflüssigkeitschromatographie gereinigt werden.

2. Verfahren zur Herstellung pharmakologisch aktiver Peptide nach Anspruch 1, dadurch gekennzeichnet, daß
A = Gln, Arg, Glu, Ser, Thr, Pro, Phe oder Tyr
B = Arg
C = Phe
D = Phe
E = Glu oder Asp
F = Ser
ist.

3. Verfahren zur Herstellung pharmakologisch aktiver Peptide nach Ansprüchen 1 und 2, dadurch gekennzeichnet, daß Prolin in Position 2 durch D-Ala² oder D-Pro² ersetzt ist.

4. Verfahren zur Herstellung pharmakologisch aktiver Peptide nach Ansprüchen 1 bis 3, dadurch gekennzeichnet, daß Thr⁴ durch eine beliebige Aminosäure ersetzt ist, außer Val, Met(O), Ile, Phe und Pro.

5. Verfahren zur Herstellung pharmakologisch aktiver Peptide nach Ansprüchen 1 bis 4, dadurch gekennzeichnet, daß die Aminosäure Prolin als Dehydroaminosäure vorliegt.

6. Verfahren zur Herstellung pharmakologisch aktiver Peptide nach Ansprüchen 1 bis 5, dadurch gekennzeichnet, daß das Tryptophan in der 3. Aminosäureposition des Peptids in der D-Form vorliegt.

7. Verfahren zur Herstellung pharmakologisch aktiver Peptide nach Ansprüchen 1 bis 6, dadurch gekennzeichnet, daß das Threonin in der 4. Position in der D-Form vorliegt.

8. Verfahren zur Herstellung pharmakologisch aktiver Peptide, dadurch gekennzeichnet, daß die jeweiligen Aminosäuren mit Hilfe eines in der Peptidchemie üblichen Syntheseapparates unter Benutzung der 9-Fluor-enylmethoxycarbonyl-Methode gekoppelt werden und anschließend mittels einer reversed phase C 18- Hochdruckflüssigkeitschromatographie gereinigt werden und die erhaltenen Peptide folgende Strukturen aufweisen:

Val-Tyr-Pro-Trp-Thr
Tyr-Pro-Trp-Thr
Tyr-Pro-Trp-Thr-Gln
Tyr-Pro-Trp-Thr-NH2
Tyr-D-Ala-Trp-Thr-OH
Tyr-D-Ala-Trp-Thr-NH2
Tyr-D-Ala-Trp-D-Thr-Tyr-OH
Tyr-D-Ala-Trp-D-Thr-Phe-OH

9. Verfahren zur Herstellung pharmakologisch aktiver Peptide nach Ansprüchen 1 bis 8, dadurch gekennzeichnet, daß diese nach Kopplung an Thyreoglobulin oder andere makromolekulare Eiweißkörper als Antigene zur Erzeugung von Antikörpern im Säugetierorganismus verwendet werden.

10. Verfahren zur Herstellung pharmakologisch aktiver Peptide nach Ansprüchen 1 bis 9, dadurch gekennzeichnet, daß Antikörper gegen die erfindungsgemäßen Peptide zur Bestimmung derselben in Körpergeweben und -flüssigkeiten verwendet werden.

## Claims (Claims for the following Contracting State(s): BE, CH, DE, FR, GB, IT, LI, LU, NL, SE)

1. Pharmacologically active peptides of the general formula: 
Tyr-Pro-Trp-Thr-T
Tyr-Pro-Trp-Thr-A-T
Tyr-Pro-Trp-Thr-A-B-T
Tyr-Pro-Trp-Thr-A-B-C-T
Tyr-Pro-Trp-Thr-A-B-C-D-T
Tyr-Pro-Trp-Thr-A-B-C-D-E-T
Tyr-Pro-Trp-Thr-A-B-C-D-E-F-T
 wherein Tyr is equal to the amino acid tyrosine which is also present as the N-terminal L-tyrosine of the general formula 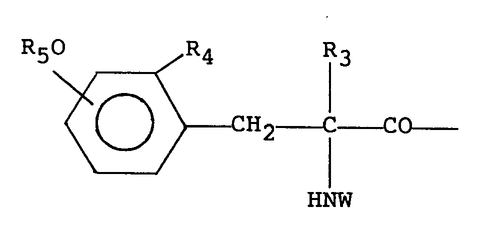 wherein the following individually signify:
R₃ represents hydrogen or an alkyl group with 1 to 4 C-atoms,
R₄ represents hydrogen or together with R₃ an ethyl bond,
R₅ represents hydrogen, an alkyl group with 1 to 4 C-atoms or a R₆CO-group,
R₆ represents a saturated or unsaturated, straight chain or branched alkyl residue with 1 to 17 C-atoms, a phenyl residue or a phenylalkyl residue with 7 to 12 C-atoms, whereby the phenyl residues can be substituted by 1 or 2 substituents from the halogen series, alkyl with 1 to 4 C-atoms or alkoxy with 1 to 4 C-atoms, whereby the R₅O-group is in the meta or para position to: 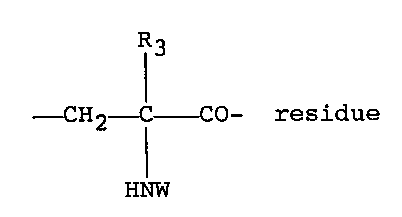 in which W represents hydrogen, alkyl with 1 to 5 C-atoms, alkenyl with 3 to 5 C-atoms, cyclopropylmethyl, cyclobutylmethyl, R₆CO-, H-Val, H-Val-Val, H-Arg, H-Lys, H-Ile, H-Tyr, H-Phe or D-Val;
Pro is equal to the amino acid proline, which is also present in the general formula 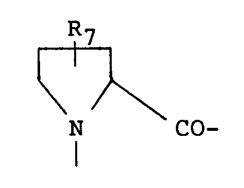 wherein the following individually signify:
R₇ represents hydrogen, a hydroxy group, an alkyl-or alkoxy group with 1 or 4 C-atoms,
- an alkyl- or alkoxy group with 1 to 4 C-atoms is substituted to the nitrogen,
- one or several keto groups are introduced into=26 the ring;
Trp is equal to the amino acid tryptophane, Thr is equal to the amino acid threonine. A, B, C, D, E and F can be any amino acid of the D- or L-form, wherein the nonapeptide Val-Val-Tyr-Pro-Trp-Thr-Gln-Arg-Phe is disclaimed; T represents OH, OR, NH₂, NHR, NR₂ or NHNHR', wherein R, if necessary, has the following significance: substituted linear or branched C₁₋₁₀-alkyl, adamantyl, C₁₋₁₀-cycloalkyl or C₆₋₈-aralkyl, preferably signifies phenyl, benzyl or phenylethyl and R' signifies hydrogen, linear or branched C₁₋₁₀-alkyl, cycloalkyl or C₆₋₈aralkyl, linear, branched or cyclic aliphatic groups C₁₋₁₆-acyl, substituted if necessary by OH, NH₂, C₁₋₁₄-alkoxy or halogen, aromatic acyl substituted if necessary by OH, NH₂, halogen or C₁₋₄-alkoxy; linear, branched or cyclic C₃₋₁₁-aliphatic urethane and their pharmaceutically acceptable salts.

2. Pharmacologically active peptides according to claim 1, characterized in that are
A = Gln, Arg, Glu, Ser, Thr, Pro, Phe or Tyr
B = Arg
C = Phe
D = Phe
E = Glu or Asp
F = Ser

3. Pharmacologically active peptides according to claims 1 and 2, characterized in that proline in the second position is substituted by D-Ala² or D-Pro².

4. Pharmacologically active peptides according to claims 1 to 3, characterized in that Thr⁴ is substituted by any amino acid, except Val, Met(O), Ile, Phe and Pro.

5. Pharmacologically active peptides according to claims 1 to 4, characterized in that the amino acid proline is present as a dehydro amino acid.

6. Pharmacologically active peptides according to claims 1 to 5, characterized in that the tryptophane in the third amino acid position of the peptide is present in the D-form.

7. Pharmacologically active peptides according to claims 1 to 6, characterized in that the threonine in the fourth position is present in the D-form.

8. Pharmacologically active peptides, characterized in that the peptides have the following structures:

Val-Tyr-Pro-Trp-Thr
Tyr-Pro-Trp-Thr
Tyr-Pro-Trp-Thr-Gln
Tyr-Pro-Trp-Thr-NH2
Tyr-D-Ala-Trp-Thr-OH
Tyr-D-Ala-Trp-Thr-NH2
Tyr-D-Ala-Trp-D-Thr-Tyr-OH
Tyr-D-Ala-Trp-D-Thr-Phe-OH

9. Pharmacologically active peptides according to claims 1 to 8, characterized in that they are used, after coupling with thyreoglobuline or other macro-molecular albumins, as antigens for the production of antibodies in the mammalian organism.

10. Pharmacologically active peptides according to claims 1 to 9, characterized in that the antibodies are used against the peptides according to this invention for the analysis of the same in body tissues and body fluids.

11. Human and animal medication, characterized in that these contain pharmacologically active peptides, and/or their derivatives, and/or their acid addition salts, and/or their metal complexes, as characterized in the claims 1 to 10.

## Claims (Claims for the following Contracting State(s): AT, ES, GR)

1. Method for the manufacture of pharmacologically active peptides characterized in that peptides of the general formula: 
Tyr-Pro-Trp-Thr-T
Tyr-Pro-Trp-Thr-A-T
Tyr-Pro-Trp-Thr-A-B-T
Tyr-Pro-Trp-Thr-A-B-C-T
Tyr-Pro-Trp-Thr-A-B-C-D-T
Tyr-Pro-Trp-Thr-A-B-C-D-E-T
Tyr-Pro-Trp-Thr-A-B-C-D-E-F-T
 are present
wherein Tyr is equal to the amino acid tyrosine which is also present as the N-terminal L-tyrosine of the general formula 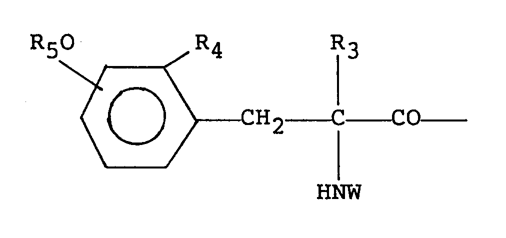 wherein the following individually signify:
R₃ represents hydrogen or an alkyl group with 1 to 4 C-atoms,
R₄ represents hydrogen or together with R₃ an ethyl bond,
R₅ represents hydrogen, an alkyl group with 1 to 4 C-atoms or a R₆CO-group,
R₆ represents a saturated or unsaturated, straight chain or branched alkyl residue with 1 to 17 C-atoms, a phenyl residue or a phenylalkyl residue with 7 to 12 C-atoms, whereby the phenyl residues can be substituted by 1 or 2 substituents from the halogen series, alkyl with 1 to 4 C-atoms or alkoxy with 1 to 4 C-atoms, whereby the R₅O-group is in the meta or para position to: 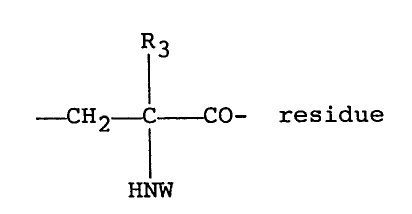 in which W represents hydrogen, alkyl with 1 to 5 C-atoms, alkenyl with 3 to 5 C-atoms, cyclopropylmethyl, cyclobutylmethyl, R₆CO-, H-Val, H-Val-Val, H-Arg, H-Lys, H-Ile, H-Tyr, H-Phe or D-Val,
Pro is equal to the amino acid proline which is also present in the general formula 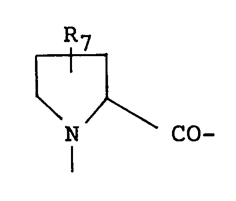 wherein the following individually signify:
R₇ represents hydrogen, a hydroxy group, an alkyl-or alkoxy group with 1 or 4 C-atoms,
- an alkyl- or alkoxy group with 1 to 4 C-atoms is substituted to the nitrogen,
- one or several keto groups are introduced into the ring;
Trp is equal to the amino acid tryptophane, Thr is equal to the amino acid threonine. A, B, C, D, E and F can be any amino acid of the D- or L-form, wherein the nonapeptide Val-Val-Tyr-Pro-Trp-Thr-Gln-Arg-Phe is disclaimed;
T represents OH, OR, NH₂, NHR, NR₂ or NHNHR', wherein R, if necessary, has the following significance: substituted linear or branched C₁₋₁₀-alkyl, adamantyl, C₁₋₁₀-cycloalkyl or C₆₋₈-aralkyl, preferably signifies phenyl, benzyl or phenylethyl and R' signifies hydrogen, linear or branched C₁₋₁₀-alkyl, cycloalkyl or C₆₋₈aralkyl, linear, branched or cyclic aliphatic groups C₁₋₁₆-acyl, substituted, if necessary, by OH, NH₂, C₁₋₁₄-alkoxy or halogen, aromatic acyl substituted if necessary, by OH, NH₂, halogen or C₁₋₄-alkoxy; linear, branched or cyclic C₃₋₁₁-aliphatic urethane and their pharmaceutically acceptable salts, the specific amino acids are coupled with the aid of an apparatus common in peptide chemistry using the method of 9-fluorenylmethoxy-carbonyl and the peptides then are purified by means of a reversed phase C18-high pressure liquid chromatography.

2. Method for the manufacture of pharmacologically active peptides according to claim 1, characterized in that are
A = Gln, Arg, Glu, Ser, Thr, Pro, Phe or Tyr
B = Arg
C = Phe
D = Phe
E = Glu or Asp
F = Ser

3. Method for the manufacture of pharmacologically active peptides according to claims 1 and 2, characterized in that proline in the second position is substituted by D-Ala² or D-Pro².

4. Method for the manufacture of pharmacologically active peptides according to claims 1 to 3, characterized in that Thr⁴ is substituted by any amino acid, except Val, Met(O), Ile, Phe and Pro.

5. Method for the manufacture of pharmacologically active peptides according to claims 1 to 4, characterized in that the amino acid proline is present as a dehydro amino acid.

6. Method for the manufacture of pharmacologically active peptides according to claims 1 to 5, characterized in that the tryptophane in the third amino acid position of the peptide is present in the D-form.

7. Method for the manufacture of pharmacologically active peptides according to claims 1 to 6, characterized in that the threonine in the fourth position is present in the D-form.

8. Method for the manufacture of pharmacologically active peptides, characterized in that the specific peptides are coupled with the aid of an apparatus common in peptide chemistry using the method of 9-fluorenylmethoxy-carbonyl and the peptides then are purified by means of a reversed phase C18-high pressure liquid chromatography and the obtained peptides show the following structure:

Val-Tyr-Pro-Trp-Thr
Tyr-Pro-Trp-Thr
Tyr-Pro-Trp-Thr-Gln
Tyr-Pro-Trp-Thr-NH2
Tyr-D-Ala-Trp-Thr-OH
Tyr-D-Ala-Trp-Thr-NH2
Tyr-D-Ala-Trp-D-Thr-Tyr-OH
Tyr-D-Ala-Trp-D-Thr-Phe-OH

9. Method for the manufacture of pharmacologically active peptides according to claims 1 to 8, characterized in that they are used, after coupling with thyreoglobuline or other macro-molecular albumins, as antigens for the production of antibodies in the mammalian organism.

10. Method for the manufacture of pharmacologically active peptides according to claims 1 to 9, characterized in that the antibodies are used against the peptides according to this invention for the analysis of the same in body tissues and body fluids.

## Revendications (Revendications pour l'(les) Etat(s) contractant(s) suivant(s): BE, CH, DE, FR, GB, IT, LI, LU, NL, SE)

1. Peptides à activité pharmacologique des formules suivantes: 
Tyr-Pro-Trp-Thr-T
Tyr-Pro-Trp-Thr-A-T
Tyr-Pro-Trp-Thr-A-B-T
Tyr-Pro-Trp-Thr-A-B-C-T
Tyr-Pro-Trp-Thr-A-B-C-D-T
Tyr-Pro-Trp-Thr-A-B-C-D-E-T
Tyr-Pro-Trp-Thr-A-B-C-D-E-F-T
 dans lesquelles
L-Tyr représente le résidu d'amino-acide L-tyrosine et aussi représente la L-tyrosine N-terminale de formule générale: 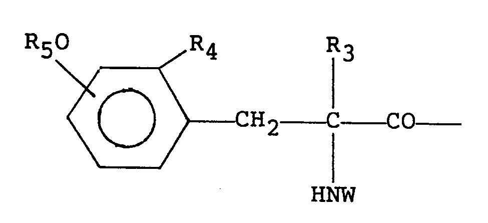 En ce formule générale représente
R₃ un atome d'hydrogène ou un groupe alkyle en C₁ à C₄,
R₄ un atome d'hydrogène ou forme avec R₃ un pont éthylène,
R₅ un atome d'hydrogène, un groupe alkyle en C₁ à C₄ ou un groupe R₆CO,
R₆ un résidu d'alkyle saturé ou non saturé, linéaire ou ramifié comportant 1 à 17 atomes de carbone, un reste phényle ou phénylalkyle comportant 7 à 12 atomes de carbone, les restes phényles pouvant être substutués par 1 ou 2 substituants de la série des halogènes, des restes alkyles en C₁ à C₄ ou d'alcoxy en C₁ à C₄, le groupe R₅0 étant en position méta ou para par rapport au reste 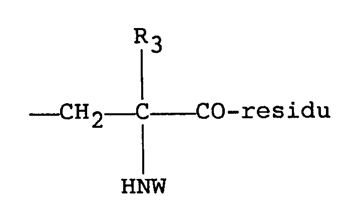 et
W étant un atome d'hydrogène, un alkyle en C₁ à C₅, un alkényle en C₃ à C₅, un cyclopropylméthyle, un cyclobutylméthyle, un groupe R₆CO-, les résidus d'amino-acides H-Val, H-Val-Val, H-Arg, H-Lys, H-Ile, H-Tyr, H-Phe ou D-Val;
Pro représente le résidu d'amino acide proline étant aussi de formule générale 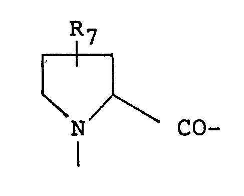 En ce formule générale représente
R₇ un atome d'hydrogène, un groupement hydroxy, un groupe alkyle ou un groupe alcoxy en C₁₋₄,
- l'atome d'azote est lié à un groupe alkyle ou un groupe alcoxy comportant 1 à 4 atomes de carbone,
- un ou plusieurs groupes céto sont introduits dans le cycle;
et
Trp représente le résidu d'amino acide tryptophane et Thr représente le résidu d'amino acide threonine.
A, B, C, D, E et F peuvent être chaques quelconques résidus d'amino acides de la configuration stéréochimique D ou L, ne pouvant pas être le nonapeptide Val-Val-Tyr-Pro-Trp-Thr-Gln-Arg-Phe.
T représente OH, OR, NH₂, NHR, NR₂ ou NHNHR', R éventuellement représentant
- un groupe alkyle en C₁₋₁₀ linéaire ou ramifié et substitué, un groupe adamantyle, un groupe cycloalkyle en C₁₋₁₀ ou un groupe aralkyle en C₆₋₈, avantageusement un groupe phényle, un groupe benzyle, ou un groupe phényléthyle,
et R' représente
- un atome d'hydrogène, un groupe alkyle en C₁₋₁₀ linéaire ou ramifié, un groupe cycloalkyle ou un groupe aralkyle en C₆₋₈, un groupement acyle en C₁₋₁₆ aliphatique, linéaire, ramifié ou cyclique, éventuellement substitué par OH, NH₂, un groupe alcoxy en C₁₋₁₄ ou halogène,
- un groupe acyle aromatique, éventuellement substitué par OH, NH₂, halogène ou un groupe alcoxy en C₁₋₄,
- un groupe de type uréthane aliphatique en C₃₋₁₁ linéaire, ramifié ou cyclique
et de même que
les sels pharmaceutiquement acceptables des substances que l'ont vient de mentionner.

2. Peptides à activité pharmacologique selon la revendication 1, caractérisés en ce que
A représente les résidus d'amino acides Gln, Arg, Glu, Ser, Thr, Pro, Phe ou Tyr,
B représente le résidu d'amino acide Arg,
C représente le résidu d'amino acide Phe,
D représente le résidu d'amino acide Phe,
E représente les résidus d'amino acides Glu ou Asp,
F représente le résidu d'amino acide Ser.

3. Peptides à activité pharmacologique selon des revendications 1 et 2, caractérisés en ce que la proline en deuxième position est remplaceé par D-Ala² ou D-Pro².

4. Peptides à activité pharmacologique suivant l'une des revendications 1-3, caractérisés en ce que la Thr⁴ est remplaceé par une amino acide chaque quelconque sauf Val, Met(O), Ile, Phe et Pro.

5. Peptides à activité pharmacologique suivant l'une des revendications 1-4, caractérisés en ce que l'amino acide proline se présente sous forme de déhydroamino-acide.

6. Peptides à activité pharmacologique, suivant l'une des revendications 1-5, caractérisés en ce que la tryptophane occupant la troisième position d'amino acide du peptide (calculé de bout N-terminal) se présente dans la configuration stéréochimique D.

7. Peptides à activité pharmacologique, suivant l'une des revendications 1-6, caractérisés en ce que la threonine occupant la quatrième position d'amino acide du peptide (calculé de bout N-terminal) se présente dans la configuration stéréochimique D.

8. Peptides à activité pharmacologique, caractérisés en ce que les peptides suivent des structures suivantes:

Val-Tyr-Pro-Trp-Thr
Tyr-Pro-Trp-Thr
Tyr-Pro-Trp-Thr-Gln
Tyr-Pro-Trp-Thr-NH2
Tyr-D-Ala-Trp-Thr-OH
Tyr-D-Ala-Trp-Thr-NH2
Tyr-D-Ala-Trp-D-Thr-Tyr-OH
Tyr-D-Ala-Trp-D-Thr-Phe-OH

9. Peptides à activité pharmacologique, suivant l'une des revendications 1-8, caractérisés en ce qu'ils peuvent appliqués comme antigenes - après couplage à thyreoglobuline ou à autres corps d'albumine macromoleculaires - à procréation des anticorps dans l'organisme de mammifère.

10. Peptides à activité pharmacologique, suivant l'une des revendications 1-9, caractérisés en ce que les anticorps peuvent appliqués contre des peptides inventives à determiner les mêmes en tissus de corps et humeurs.

11. Préparations pharmaceutiques, caracterisées en ce qu'elles contiennent des peptides à activité pharmacologique et/ou leurs dérivés et/ou leurs sels d'acide et/ou leurs complexes métallique qu'ils sont definés en revendications 1-10.

## Revendications (Revendications pour l'(les) Etat(s) contractant(s) suivant(s): AT, ES, GR)

1. Procédé de préparation de peptides à activité pharmacologique des formules suivantes: 
Tyr-Pro-Trp-Thr-T
Tyr-Pro-Trp-Thr-A-T
Tyr-Pro-Trp-Thr-A-B-T
Tyr-Pro-Trp-Thr-A-B-C-T
Tyr-Pro-Trp-Thr-A-B-C-D-T
Tyr-Pro-Trp-Thr-A-B-C-D-E-T
Tyr-Pro-Trp-Thr-A-B-C-D-E-F-T
 dans lesquelles
L-Tyr représente le résidu d'amino-acide L-tyrosine et aussi représente la L-tyrosine N-terminale de formule générale: 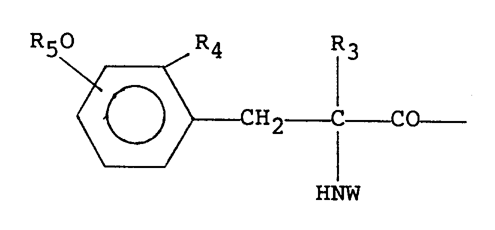 En ce formule générale représente
R₃ un atome d'hydrogène ou un groupe alkyle en C₁ à C₄,
R₄ un atome d'hydrogène ou forme avec R₃ un pont éthylène,
R₅ un atome d'hydrogène, un groupe alkyle en C₁ à C₄ ou un groupe R₆CO,
R₆ un résidu d'alkyle saturé ou non saturé, linéaire ou ramifié comportant 1 à 17 atomes de carbone, un reste phényle ou phénylalkyle comportant 7 à 12 atomes de carbone, les restes phényles pouvant être substutués par 1 ou 2 substituants de la série des halogènes, des restes alkyles en C₁ à C₄ ou d'alcoxy en C₁ à C₄, le groupe R₅0 étant en position méta ou para par rapport au reste 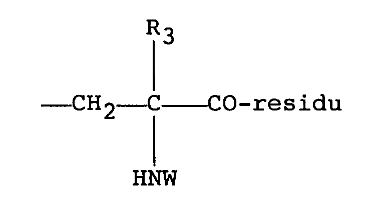 et
W étant un atome d'hydrogène, un alkyle en C₁ à C₅, un alkényle en C₃ à C₅, un cyclopropylméthyle, un cyclobutylméthyle, un groupe R₆CO-, les résidus d'amino-acides H-Val, H-Val-Val, H-Arg, H-Lys, H-Ile, H-Tyr, H-Phe ou D-Val;
Pro représente le résidu d'amino acide proline étant aussi de formule générale 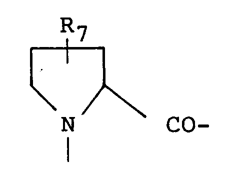 En ce formule générale représente
R₇ un atome d'hydrogène, un groupement hydroxy, un groupe alkyle ou un groupe alcoxy en C₁₋₄,
- l'atome d'azote est lié à un groupe alkyle ou un groupe alcoxy comportant 1 à 4 atomes de carbone,
- un ou plusieurs groupes céto sont introduits dans le cycle;
et
Trp représente le résidu d'amino acide tryptophane et Thr représente le résidu d'amino acide threonine.
A, B, C, D, E et F peuvent être chaques quelconques résidus d'amino acides de la configuration stéréochimique D ou L, ne pouvant pas être le nonapeptide Val-Val-Tyr-Pro-Trp-Thr-Gln-Arg-Phe.
T représente OH, OR, NH₂, NHR, NR₂ ou NHNHR', R éventuellement représentant
- un groupe alkyle en C₁₋₁₀ linéaire ou ramifié et substitué, un groupe adamantyle, un groupe cycloalkyle en C₁₋₁₀ ou un groupe aralkyle en C₆₋₈, avantageusement un groupe phényle, un groupe benzyle, ou un groupe phényléthyle,
et R' représente
- un atome d'hydrogène, un groupe alkyle en C₁₋₁₀ linéaire ou ramifié, un groupe cycloalkyle ou un groupe aralkyle en C₆₋₈, un groupement acyle en C₁₋₁₆ aliphatique, linéaire, ramifié ou cyclique, éventuellement substitué par OH, NH₂, un groupe alcoxy en C₁₋₁₄ ou halogène,
- un groupe acyle aromatique, éventuellement substitué par OH, NH₂, halogène ou un groupe alcoxy en C₁₋₄,
- un groupe de type uréthane aliphatique en C₃₋₁₁ linéaire, ramifié ou cyclique
et de même que
les sels pharmaceutiquement acceptables des substances que l'ont vient de mentionner, en couplant les acides aminés choisis sous l'aide d'un appareil habituel pour la synthèse dans la chimie des peptides en utilisant la méthode de 9-fluorenylmethoxycarbonyle et aprés on purifie les peptides obtenus par une reversed phase C18-chromatographie en phase liquide à pression élevée (HPLC).

2. Procédé de préparation de peptides à activité pharmacologique selon la revendication 1, caractérisé en ce que
A représente les résidus d'amino acides Gln, Arg, Glu, Ser, Thr, Pro, Phe ou Tyr,
B représente le résidu d'amino acide Arg,
C représente le résidu d'amino acide Phe,
D représente le résidu d'amino acide Phe,
E représente les résidus d'amino acides Glu ou Asp,
F représente le résidu d'amino acide Ser.

3. Procédé de préparation de peptides à activité pharmacologique selon des revendications 1 et 2, caractérisé en ce que la proline en deuxième position est remplaceé par D-Ala² ou D-Pro².

4. Procédé de préparation de peptides à activité pharmacologique suivant l'une des revendications 1-3, caractérisé en ce que la Thr⁴ est remplaceé par une amino acide chaque quelconque sauf Val, Met(O), Ile, Phe et Pro.

5. Procédé de préparation de peptides à activité pharmacologique suivant l'une des revendications 1-4, caractérisé en ce que l'amino acide proline se présente sous forme de déhydroamino-acide.

6. Procédé de préparation de peptides à activité pharmacologique, suivant l'une des revendications 1-5, caractérisé en ce que la tryptophane occupant la troisième position d'amino acide du peptide (calculé de bout N-terminal) se présente dans la configuration stéréochimique D.

7. Procédé de préparation de peptides à activité pharmacologique, suivant l'une des revendications 1-6, caractérisé en ce que la threonine occupant la quatrième position d'amino acide du peptide (calculé de bout N-terminal) se présente dans la configuration stéréochimique D.

8. Procédé de préparation de peptides à activité pharmacologique, caractérisé en ce qu'on couple les acides aminés choisis sous l'aide d'un appareil habituel pour la synthèse dans la chimie des peptides en utilisant la méthode de 9-fluorenylmethoxycarbonyl et aprés on purifie les peptides obtenus par une reversed phase C18-chromatographie en phase liquide à pression élevée (HPLC) et lesquels peptides obtenus suivent des structures suivantes:

Val-Tyr-Pro-Trp-Thr
Tyr-Pro-Trp-Thr
Tyr-Pro-Trp-Thr-Gln
Tyr-Pro-Trp-Thr-NH2
Tyr-D-Ala-Trp-Thr-OH
Tyr-D-Ala-Trp-Thr-NH2
Tyr-D-Ala-Trp-D-Thr-Tyr-OH
Tyr-D-Ala-Trp-D-Thr-Phe-OH

9. Procédé de préparation de peptides à activité pharmacologique, suivant l'une des revendications 1-8, caractérisé en ce qu'ils peuvent appliqués comme antigenes - après couplage à thyreoglobuline ou à autres corps d'albumine macromoleculaires - à procréation des anticorps dans l'organisme de mammifère.

10. Procédé de préparation de peptides à activité pharmacologique, suivant l'une des revendications 1-9, caractérisé en ce que les anticorps peuvent appliqués contre des peptides inventives à determiner les mêmes en tissus de corps et humeurs.
